# EUROPÄISCHE PATENTSCHRIFT

(11) **EP 1 052 955 B1**
(45) Veröffentlichungstag und Bekanntmachung des Hinweises auf die Patenterteilung: **03.03.2004**
(21) Anmeldenummer: 99947343.2
(22) Anmeldetag: 14.09.1999
(51) Int. Cl.: A61F 5/02

(54) **BANDAGE FÜR KÖRPERTEILE**
BANDAGE FOR PARTS OF THE BODY
BANDAGE POUR PARTIE CORPORELLE

(30) Priorität: 03.12.1998 DE 19855923
(43) Veröffentlichungstag der Anmeldung: 22.11.2000
(73) Patentinhaber: Bauerfeind Orthopädie GmbH & Co. KG, 47880 Kempen (DE)
(72) Erfinder: REINHARDT, Holger, C., W., D-47906 Kempen (DE); BAUERFEIND, Hans, B., D-47906 Kempen (DE)
(74) Vertreter: Bardehle, Heinz, Dipl.-Ing.
(86) Internationale Anmeldenummer: PCT/EP1999/006794
(87) Internationale Veröffentlichungsnummer: WO 2000/032139

(56) Entgegenhaltungen:
- WO-A-94/09728
- DE-A- 4 337 354
- US-A- 3 096 760
- US-A- 5 500 959
- US-A- 5 785 671

## Beschreibung

Die Erfindung bezieht sich auf Bandage für Körperteile, die in einem Überlappungsbereich durch zwei an der Bandage mit jeweils ihrem einen Ende in symmetrischer Anordnung befestigte Spannbänder zusammenziehbar ist, deren jeweils anderes Ende als Zugende an der Bandage stramm festlegbar ist.

Eine derartige Bandage ist in der DE OS 43 37 354 offenbart. Die bekannte Bandage besteht aus einem Bauchgurt, der über eine Fixierplatte geschlossen wird, die zur Stützung der Rückenpartie des Patienten dient. Die Verbindung zwischen der Fixierplatte und dem Bauchgurt wird durch Spannbänder hergestellt, die jeweils an den Enden des Bauchgurtes befestigt und durch Öffnungen in der Fixierplatte geschlungen sind. Durch Ziehen an den freien Enden der Spannbänder wird dann die Bandage auf den betreffenden Körperumfang eingestellt. Die freien Enden der Spannbänder werden dann mittels Verschlusselementen an dem Bauchgurt befestigt.

Eine Bandage, bei der ein ähnliches Prinzip der Spannung der Bandage angewendet wird, ist in der WO 96/32079 A1 offenbart. Bei dieser Bandage wird aus anatomischen Gründen zur Erfassung der jeweiligen Körperkontur ein Spannband in drei Einzelbänder aufgefächert, die durch drei Ösen ein Mal umgelenkt werden, wodurch erreicht wird, dass die auf die Einzelbänder wirkende Zugkraft um 180° umgelenkt wird. Im Gegensatz zu der Bandage gemäß der DE OS 43 37 354, bei der nach Umlenkung der Spannbänder diese mit ihren Zugenden nach außen gezogen werden, weist die Zugrichtung am Zugende des Spannbandes gemäß der WO 96/32079 A1 nach innen hin.

Zum Anbringen derartiger Bandagen ist eine erhebliche Zugkraft aufzubringen, insbesondere wenn die Bandage eine besonders starke Stützfunktion ausüben soll. Es ist bekannt, dass zum Anbringen solcher Bandagen oft eine weitere Person Hilfe leisten muss, da der betreffende Patient aufgrund seiner gesundheitlichen Situation nicht in der Lage ist, eine ausreichende Zugkraft auf die Spannbänder auszuüben.

Eine unsymmetrisch zusammengezogene Bandage, bei der sich die Bandage in einem Überlappungsbereich zusammenschiebt, ist in der US-PS 5,500,959 offenbart. Über den Überlappungsbereich ist ein flaschenzugartig geführtes Spannband gelegt, dessen Ende durch ein Schloss geführt ist, durch das mit Andrücken eines Schlosshebels das Spannband eingeklemmt und damit das Spannband am Zurückgleiten gehindert wird. Die bekannte Bandage weist damit von dem Prinzip symmetrisch angeordneter Spannbänder weg, bei ihrem Anlegen und Festziehen des Spannbandes entsteht im Übrigen eine in Umfangsrichtung wirkende Kraft, durch die die Bandage an dem betreffenden Körperteil beim Anziehen des Spannbandes zum Verrutschen neigt. Dieser Effekt wird bei der der Erfindung zugrundeliegenden Bandage mit symmetrischer Anordnung der Spannbänder vermieden.

Der Erfindung liegt die Aufgabe zugrunde, das Anlegen der eingangs erwähnten Bandage wesentlich zu erleichtern, ohne dass dabei von dem betreffenden Patienten ein besonderer Kraftaufwand zu leisten ist. Erfindungsgemäß geschieht dies dadurch, dass beiderseits des Überlappungsbereichs je ein aus elastischem Material bestehender Kontraktionsbereich anschließt und jedes Spannband durch Umlenkringe, die mit Abstand voneinander unter Einschluss jeweils eines Kontraktionsbereichs an der Bandage befestigt sind, derart hin- und hergehend geführt ist, dass Zugkräfte, die auf die aufeinander zu gerichteten Zugenden der Spannbänder ausgeübt werden, nach Art eines Flaschenzuges die Kontraktionsbereiche als Verkürzung der Bandage zusammenziehen.

Aufgrund der Anordnung der Umlenkringe jeweils im Zusammenhang mit den von ihnen eingeschlossenen Kontraktionsbereichen innerhalb der Bandage ergibt sich eine Hin- und Herführung der die Bandage zusammenziehenden Spannbänder, die nach Art eines Flaschenzuges wirkt und damit eine hohe Zugkraft auf die Bandage ausüben kann, ohne dass der betreffende Patient hierfür eine besondere Anstrengung aufbieten muss. Durch die erfindungsgemäße Konstruktion der Bandage mit zwei den Überlappungsbereich einschließenden Kontraktionsbereichen und der die Kontraktionsbereiche jeweils einschließende Hin- und Herführung der Spannbänder wird das Flaschenzugprinzip in eleganter Weise in die Bandage integriert, wodurch diese auch für schwächere Patienten ohne Hilfe von Drittpersonen anlegbar ist. Dabei ergibt sich noch der besondere Vorteil, dass der Patient in der Lage ist, den von der Bandage ausgeübten Radialdruck durch seine Zugkraft leicht einstellen zu können, da der Flaschenzugeffekt sich als Übersetzung der auf die Spannbänder ausgeübten Zugkraft auf den auf die Bandage ausgeübten Zug auswirkt.

Den Teil der Bandage neben dem Kontraktionsbereich gestaltet man zweckmäßig aus einem weniger elastischen, insbesondere unelastischen Material. Dies hat den Vorteil, dass beim Anziehen der Bänder sich der Kontraktionsbereich aufgrund seiner Elastizität zusammenziehen kann, ohne dass es dabei im Kontraktionsbereich zu einer Faltenbildung kommen kann.

Grundsätzlich genügt es, wenn die Bandage mit nur einem Kontraktionsbereich und den zugehörigen Spannbändern versehen wird. Es ist jedoch zweckmäßig, die Bandage so zu gestalten, daß der Kontraktionsbereich mit zugehörigem Spannband bzw. Spannbändern zweimal in symmetrischer Anordnung vorhanden ist. In diesem Falle kann der Patient mit jeweils einer Hand an den zu einem Kontraktionsbereich gehörenden Spannband bzw. Spannbändern ziehen und damit den Kontraktionsbereich entsprechend zusammenziehen, was bei zwei Kontraktionsbereichen in symmetrischer Anordnung dann zu einer entsprechend hohen Kontraktion führt, die sich bei Anlegen der Bandage symmetrisch am Körper seitlich an den beiden Hüften abspielt.

In den Figuren sind Ausführungsbeispiele der Erfindung dargestellt. Es zeigen:
- Fig. 1: die Bandage in perspektivischer Sicht mit zwei Kontraktionsbereichen in symmetrischer Anordnung,
- Fig. 2: eine schematische Darstellung der Spannbandführung bei einem Kontraktionsbereich gemäß Figur 1,
- Fig. 3: eine Abwandlung der Spannbandführung in der Weise, daß gegenüber der Darstellung nach Fig. 2 weitere Umlenkungen im Sinne der Vervielfachung des Flaschenzugprinzips vorgesehen sind,
- Fig. 4: eine Abwandlung der Gestaltung gemäß Fig. 3.

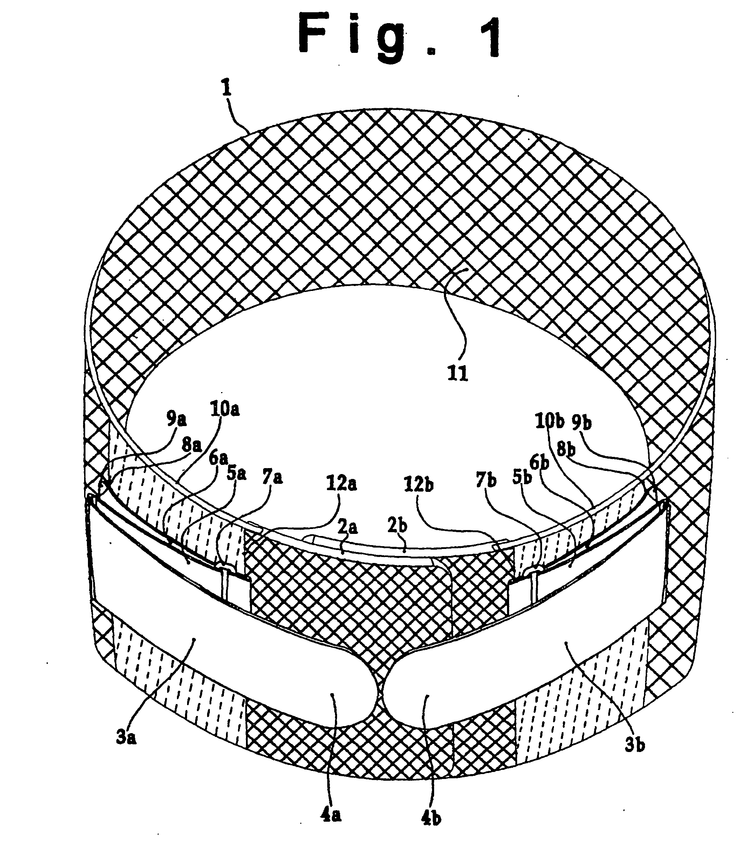

In Figur 1 ist die Bandage 1 in perspektivischer Sicht dargestellt. Sie wird durch die beiden Überlappungsbereiche 2a und 2b zu einem Ring geschlossen. Die beiden Überlappungsbereiche 2a und 2b sind auf ihren einander zugewandten Seiten nach Art eines Klettverschlusses ausgebildet, so daß je nach Größe des zu behandelnden Körperteils die Überlappungsbereiche 2a und 2b mehr oder minder weit übereinander gelegt werden können, in welcher Lage sie dann durch den zwischen ihnen vorgesehenen Klettverschluß gegenseitig befestigt werden.

Es sei jedoch darauf hingewiesen, daß die Bandage 1 natürlich auch aus einem einzigen durchgehenden Materialstück hergestellt sein kann, das dann allerdings nicht in besonders einfacher Weise an die jeweilige Größe des betreffenden Körperteils anpaßbar ist.

Neben den beiden Überlappungsbereichen 2a und 2b sind die Kontraktionsbereiche 10a und 10b angeordnet, die in den hinteren Rundungsteil 11 der Bandage 1 übergehen. Die Kontraktionsbereiche bestehen aus einem elastischen Textilmaterial, wogegen es sich bei dem übrigen Teil der Bandage, also den beiden Überlappungsbereichen 2a und 2b und dem Rundungsteil 11 um ein im wesentlichen unelastisches Textilmaterial handelt.

Im Bereich des Übergangs von dem Kontraktionsbereich 10a, 10b zu der hinteren Rundung 11 ist ein Ende 9a, 9b des Spannbandes 3a, 3b befestigt. Von diesem Ende 9a, 9b erstreckt sich die Länge 6a, 6b des Spannbandes 3a, 3b zu dem Umlenkring 7a, 7b, von dem aus das Spannband 3a, 3b über die Länge 5a, 5b zu dem weiteren Umlenkring 8a, 8b geführt ist. Aufgrund einer weiteren Umlenkung des Spannbandes 3a, 3b durch den Umlenkring 8a, 8b gelangt dann das Spannband 3a, 3b mit seinem Zugende 4a, 4b in den vorderen mittleren Bereich der Bandage 1, also zu dem Überlappungsbereich 2a, an dem das Zugende 4a, 4b festgelegt werden kann. Hierzu dient ein zwischen dem Zugende 4a, 4b und der Außenseite des Überlappungsbereiches 2a angeordneter Klettverschluß.

Nach Anlegen der Bandage 1, z. B. um den Hüftbereich eines Patienten, und Schließen der Überlappungsbereiche 2a und 2b kann der Patient an den Zugenden 4a, 4b der Spannbänder 3a, 3b mit jeweils einer Hand ziehen. Wenn er dabei die Bandage 1 so angelegt hat, daß die Überlappungsbereiche 2a und 2b auf der Körperseite mit dem Bauchnabel liegen, dann lassen sich von dem Patienten die beiden Zugenden 4a und 4b mit jeweils einer Hand leicht erfassen und nach vom ziehen. Aufgrund der bei dem dargestellten Ausführungsbeispiel gewählten zweimaligen Umlenkung pro Spannband 3a, 3b über die Umlenkringe 7a und 8a bzw. 7b und 8b ergibt sich eine vom Flaschenzug her bekannte größere Zuglänge am jeweiligen Zugende 4a, 4b gegenüber der Annäherung der beiden Umlenkringe 7a und 8a bzw. 7b und 8b, was sich auf eine entsprechende Verstärkung des Zuges von dem Umlenkring 7a, 7b auf die Länge 6a, 6b des Spannbandes 3a, 3b auswirkt. Aufgrund der Befestigung des Umlenkringes 7a, 7b an der Übergangsstelle 12a, 12b vom Kontraktionsbereich 10a, 10b zum Überlappungsbereich 2a bzw. 2b ergibt sich ein Zusammenziehen des Kontraktionsbereichs 10a, 10b, womit die Bandage 1 entsprechend stramm um das entsprechende Körperteil, hier also um die Hüftpartie des Patienten, gezogen wird. Nach derartig erfolgtem Zusammenziehen des Kontraktionsbereichs 10a, 10b werden dann die Enden 4a, 4b der Spannbänder 3a, 3b mittels des oben erwähnten Klettverschlusses an der Außenseite des Überlappungsbereiches 2a festgelegt, womit die Bandage 1 ihren strammen Sitz an dem betreffenden Körperteil beibehält.

Die einerseits an dem Bereich 9a, 9b und den Übergangsstellen 12a, 12b befestigten Umlenkringe 8a, 8b und 7a, 7b schließen dabei jeweils den Kontraktionsbereich 10a, 10b zwischen sich ein, so daß die beim Ziehen an den Zugenden 4a, 4b aufgebrachte Zugkraft sich verstärkt auf das Zusammenziehen des Kontraktionsbereichs 10a, 10b auswirkt.

Die Spannbänder 3a, 3b sind aus im wesentlichen unelastischen Textilmaterial gefertigt. Sie können natürlich auch eine gewisse Elastizität aufweisen, jedoch ist dies für die richtige Wirkungsweise der erfindungsgemäßen Bandage nicht erforderlich. Die Umlenkringe 7a, 7b, 8a, 8b sind hier als längliche Ösen gestaltet, um die flachen Spannbänder 3a, 3b günstig zu fuhren. Anstelle jeweils eines Spannbandes 3a, 3b mit einem Kontraktionsbereich 10a, 10b können natürlich auch mehrere Spannbänder an einem Kontraktionsbereich angeordnet sein, was insbesondere dann günstig ist, wenn es sich um eine relativ breite Bandage handelt. Längliche Umlenkringe sind beispielsweise in der eingangs erwähnten DE OS 43 37 354 dargestellt.

Bei dem in Figur 1 dargestellten Ausführungbeispiel ist bewußt eine symmetrische Anordnung von zwei Kontraktionsbereichen 10a und 10b vorgesehen. Dies ist besonders für das Anlegen der Bandage 1 mit zwei Händen von Vorteil. Es sei jedoch darauf hingewiesen, daß natürlich die Bandage auch mit nur einem Kontraktionsbereich ausgestattet werden kann, der insbesondere bei Bandagen für kleinere Körperteile als einziger Kontraktionsbereich ausreichen kann.

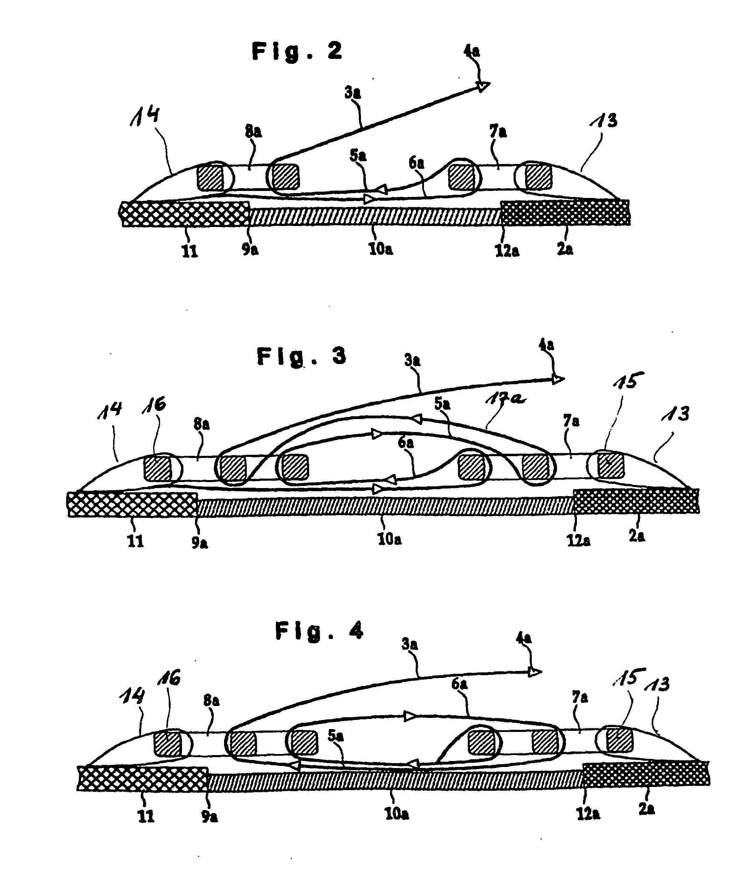

Figur 2 zeigt in schematischer Darstellung in einer Ansicht auf die Kante der Bandage 1 den Kontraktionsbereich 10a, der einerseits in die Rundung 11 und andererseits in den Überlappungsbereich 2a übergeht. Die beiden Umlenkringe 7a und 8a sind im Schnitt gezeichnet. Sie dienen einerseits zur Umlenkung des Spannbandes 3a mit seinen Längen 5a und 6a und andererseits zur Befestigung des Umlenkringes 8a an dem Material der Rundung 11 in der Nähe des Endes 9a der Bandage 3a (Schlaufe 14) und der Befestigung des Umlenkrings 7a in der Nähe des Übergangsbereiches 12a zwischen Kontraktionsbereich 10a und dem Überlappungsbereich 2a (Schlaufe 13).

Aus Figur 2 ist besonders deutlich ersichtlich, wie beim Anziehen des Zugendes 4a in der eingezeichneten Pfeilrichtung die Länge 5a folgt und dabei den Umlenkring 7a dichter an den Umlenkring 8a heranzieht, wobei sich die Länge 6a entsprechend verkürzt, womit der Kontraktionsbereich 10a entsprechend zusammengezogen wird.

Bei dem in der Figur 1 dargestellten Ausführungsbeispiel ist das Spannband 3a, 3b über eine zweimalige Umlenkung geführt, nämlich mittels der Umlenkringe 7a, 7b und 8a, 8b. Es ist auch möglich, das Spannband jeweils über mehr als zwei Umlenkungen zu führen, um damit im Sinne der Flaschenzugwirkung eine weitere Verstärkung der Zugwirkung bei Anziehen der Zugenden der Spannbänder zu ermöglichen.

In der Figur 3 ist eine Darstellung ähnlich derjenigen gemäß Figur 2 gezeigt, die in einem Ausschnitt aus der Bandage (wie auch in Fig. 2 dargestellt) den Kontraktionsbereich 10a sowie im Ausschnitt die Rundung 11 und den Überlappungsbereich 2a zeigt. Zusätzlich zu den beiden in Fig. 2 dargestellten Längen 5a und 6a ist bei der Ausführungsform gemäß Fig. 3 die weitere Länge 17a vorgesehen, so daß sich insgesamt vier Umlenkungen ergeben, durch die die Flaschenzugwirkung beim Ziehen an dem Zugende 4a eine entsprechende Zusammenziehungskraft auf den Kontraktionsbereich 10a ausübt. Die Umlenkringe 7a und 8a sind für diese Gestaltung jeweils mit drei Sprossen versehen, wobei die äußere Sprosse 15 bzw. 16 zur Befestigung über die Schlaufen 14 und 15 dient.

In Fig. 4 ist eine Abwandlung der Gestaltung gemäß Fig. 3 dargestellt, bei der ebenfalls mehrere Umlenkungen stattfinden, wobei das Spannband 3a ähnlich wie bei der Gestaltung gemäß Fig. 2 in die beiden Längen 5a und 6a aufgeteilt ist, dabei aber dreimal umgelenkt wird, wodurch ebenfalls gegenüber der Anordnung gemäß Fig. 2 eine Verstärkung der Anziehungskraft bewirkt wird, die den Kontraktionsbereich 10a zusammenzieht.

## Patentansprüche

1. Bandage (1) für Körperteile, die in einem Überlappungsbereich (2a, 2b) durch zwei an der Bandage (1) mit jeweils ihrem einen Ende (9a, 9b) in symmetrischer Anordnung befestigte Spannbänder (3a, 3b) zusammenziehbar ist, deren jeweils anderes Ende als Zugende (4a, 4b) an der Bandage (1) stramm festlegbar ist, **dadurch gekennzeichnet, dass** beiderseits des Überlappungsbereichs (2a, 2b) je ein aus elastischem Material bestehender Kontraktionsbereich anschließt und jedes Spannband (3a, 3b) durch jeweils zwei Umlenkringe (7a, 7b; 8a, 8b), die mit Abstand voneinander unter Einschluss jeweils eines Kontraktionsbereichs (10a, 10b) an der Bandage befestigt sind, derart hin- und hergehend geführt ist, dass Zugkräfte, die auf die aufeinander zu gerichteten Zugenden (4a, 4b) der Spannbänder (3a, 3b) ausgeübt werden, nach Art eines Flaschenzuges die Kontraktionsbereiche (10a, 10b) als Verkürzung der Bandage zusammenziehen.

2. Bandage nach Anspruch 1, **dadurch gekennzeichnet, dass** der Teil der Bandage (1) neben den Kontraktionsbereichen (10a, 10b) aus einem wesentlich weniger elastischen, insbesondere unelastischen Material besteht.

## Claims

1. Bandage (1) for parts of the body, which bandage can be drawn tight in an overlap area (2a, 2b) by means of two tightening straps (3a, 3b) whose one end (9a, 9b) is in each case secured in a symmetrical arrangement on the bandage (1) and whose other end can in each case be fixed firmly on the bandage (1) as a tensioning end (4a, 4b), **characterized in that** on both sides of the overlap area (2a, 2b) there is in each case a contraction area made of an elastic material, and each tightening strap (3a, 3b) is guided back and forth through two deflection rings (7a, 7b; 8a, 8b), which are secured on the bandage at a distance from one another, with in each case inclusion between them of a contraction area (10a, 10b), in such a way that tensile forces exerted on the tensioning ends (4a, 4b) of the tightening straps (3a, 3b) directed towards one another act in the manner of a pulley and draw the contraction areas (10a, 10b) together and so shorten the bandage.

2. Bandage according to Claim 1, **characterized in that** the part of the bandage (1) next to the contraction areas (10a, 10b) is made of a substantially less elastic material, in particular a nonelastic material.

## Revendications

1. Bandage (1) destiné à des parties corporelles, pouvant être serré dans une zone de recouvrement (2a, 2b) par deux bandes de tension (3a, 3b) fixées par l'une de leurs extrémités respectives (9a, 9b) au bandage (1) dans une disposition symétrique, dont l'autre extrémité respective peut être fixée de façon tendue sur le bandage (1) en tant qu'extrémité de traction (4a, 4b), **caractérisé en ce qu'**une zone de contraction constituée d'une matière élastique se raccorde aux deux côtés de la zone de recouvrement (2a, 2b), et **en ce que** chaque bande de tension (3a, 3b) est guidée en va-et-vient par deux anneaux de déviation (7a, 7b ; 8a, 8b), qui sont fixés au bandage à un certain écartement les uns des autres en incluant respectivement une zone de contraction (10a, 10b), de telle sorte que des forces de traction, qui sont exercées sur les extrémités de traction (4a, 4b) des bandes de tension (3a, 3b) orientées l'une vers l'autre, resserrent les zones de contraction (10a, 10b) à la manière d'un moufle à titre de raccourcissement du bandage.

2. Bandage selon la revendication 1, **caractérisé en ce que** la partie du bandage (1) située à côté des zones de contraction (10a, 10b) est constituée d'une matière nettement moins élastique, notamment non élastique.
